# EUROPEAN PATENT SPECIFICATION

(11) **EP 1 009 483 B1**
(45) Date of publication and mention of the grant of the patent: **21.12.2005**
(21) Application number: 98945776.7
(22) Date of filing: 25.08.1998
(51) Int. Cl.: A61N 5/00, A61N 5/06

(54) **TREATMENT DEVICE FOR TOPICAL PHOTODYNAMIC THERAPY**
EINRICHTUNG ZUR TOPISCHEN PHOTODYNAMISCHEN THERAPIE
DISPOSITIF DE TRAITEMENT POUR THERAPIE PHOTODYNAMIQUE TOPIQUE

(30) Priority: 25.08.1997 US 57356 P
(43) Date of publication of application: 21.06.2000
(73) Proprietor: Advanced Photodynamic Technologies, Inc., Mendota Heights, MN 55118 (US)
(72) Inventor: BIEL, Merrill, A., Mendota Heights, MN 55118 (US)
(74) Representative: Kenyon, Sarah Elizabeth
(86) International application number: PCT/US1998/017589
(87) International publication number: WO 1999/010046

(56) References cited:
- WO-A-97/04836
- WO-A-98/06456
- US-A- 4 234 907
- US-A- 4 646 743
- US-A- 4 761 047
- US-A- 4 852 549
- US-A- 5 445 608
- US-A- 5 616 140
- US-A- 5 766 234
- PATENT ABSTRACTS OF JAPAN vol. 1996, no. 01, 31 January 1996 (1996-01-31) & JP 07 240536 A (SHIMADZU CORP), 12 September 1995 (1995-09-12)

## Description

### Background of the Invention

The invention relates to a medical device for photodynamic therapy (PDT). More specifically, the invention relates to a generally flexible or rigid conforming patch or pad and a shaped article such as a mouthpiece which provide light sources for topical PDT. The present invention advantageously uses light energy to treat or detect pathologies of living tissue, including cancer and microbiological pathogens. The present invention may be used in combination with photosensitizing agents.

United States Patent No. 4,822,335, entitled, *Apparatus For Treatment Of Cancer With Photodiode*, purportedly discloses an apparatus for the treatment of a cancerous lesion part by irradiating a light energy from a light source to the cancerous lesion part having absorbed and accumulated in advance therein a photosensitive substance with an affinity for tumors. The light source comprises a first diode adapted to excite the photosensitive substance from the ground state to a singlet state of higher energy level and a second photodiode adapted to excite an energy level of the photosensitive substance which has transited from the singlet state to a triplet state to a still higher energy level.

United States Patent No. 5,358,503, entitled, *Photo-Thermal Therapeutic Device and Method,* purportedly discloses an apparatus for simultaneous or selective treatment of an area of the skin and adjacent subcutaneous structure of a patient utilizing photo energy and therapeutic heat, which includes a plurality of juxtaposed diodes. Each diode has a longitudinal axis and is capable of projecting a non-coherent cone of light which overlaps the cone of light from each juxtaposed diode so that the light completely covers the treatment area. A pad or appliance holds the diodes in juxtaposed position with each other.

United States Patent No. 5,611,793, entitled, *Laser Treatment,* purportedly discloses a method of disinfecting or sterilizing tissues of the oral cavity or a wound or lesion in the oral cavity. The method includes applying a photosensitising compound to the tissue and irradiating with laser light at a wavelength absorbed by the photosensitising compound.

WO 97/04836 to Dusa Pharmaceuticals Inc., discloses a light emitting patch for PDT comprising a hydrogel layer providing optical coupling between light emitting members and a skin surface and providing a medium through which photopharmaceuticals may be dispersed.

US 5616140 to Prescott, M discloses a light emitting treatment device comprising an array of light emitting members mounted on a flexible circuit board within a flexible housing. Each light emitting member has an output of around 2.6 mW and a heat sink is provided in the housing.

US 5445608 to Chen et al. discloses a light emitting implantable probe. The probe may be flexible and comprises an array of light emitting members connected to a central bar and enclosed in a polymer material. The central bar may be used to disperse heat.

### Summary of the Invention

The invention relates to a PDT treatment device which is configured to deliver light energy to a treatment site from a plurality of light emitting members disposed in the device.

According to an aspect of the present invention, there is provided a light emitting treatment device characterised by a flexible substrate element comprising a gel material and adapted to be used in association with a treatment field; one or more light emitting members disposed within the substrate element, said one or more light emitting members providing a light dosage rate to the treatment field at a value between about 0 mW/cm² to about 150 mW/cm²; a heat dissipating layer for transferring heat generated by the one or more light emitting members away from the treatment field; and an outer layer disposed upon the substrate element for restricting the area of the treatment field exposed to light from the treatment device.

The treatment device is flexible and is used for wound treatment and biostimulation. The treatment device with light emitting members operates at wavelengths ranging from about 450nm to about 850nm; a dosage rate ranging from about 0 mW/cm² to about 150 mW/cm²; and a light dose ranging from 0 to 300 J/cm². The treatment device may have surface monitor capabilities which include isotropic or anisotropic light detectors or photodetectors. The light detectors may be used in combination with a controller to automatically monitor light dosage, light power and dosage rate.

In a preferred embodiment, a surface-conforming covering such as a light-diffusing pad and gel liner contain an integrated array of vertical cavity surface emitting lasers (VCSEL) as a light emitting source for topical PDT treatment.

In another embodiment, a shaped article is configured to direct energy and is used for PDT treatment. One embodiment of the shaped article includes a mouthpiece for covering and treating a patient's gumline. Another embodiment includes a shaped article for treatment of the roof of a mouth.

In sum, an embodiment of the invention relates to a light emitting treatment device including a shell and liner. The liner is at least partially disposed in the shell. One or more light emitting members are disposed in at least one of the liner or the shell. The shell, liner material and light emitting members form an assembly. The light emitting members are configured to emit energy from the assembly for photodynamic therapy of a body surface. The shell and liner may be made of a polymeric material. The shell or the liner may be made of a gel. The shell and the liner may be configured in a substantially integral structure. The treatment device is substantially flexible and may conform to a body surface. The light emitting members may be disposed in a substantially uniform array. The light emitting members may be configured to emit energy in a substantially uniform pattern. The light emitting members may be configured in a light diffusing layer. The light emitting treatment device may further include a heat dissipating layer, a layer of gold or gold alloy, or a layer of adhesive disposed on at least one surface of the shell or liner.

The invention also relates to a light emitting treatment device including a shell made of a polymeric material and a liner made of a polymeric material including a gel. The liner is at least partially disposed in the shell. One or more light emitting members are disposed in an array in at least one of the liner or the shell. The shell, liner material and the light emitting members form an assembly. The light emitting members are configured to emit energy from the assembly in a substantially uniform pattern for photodynamic therapy. A heat dissipating layer is associated with the assembly. A layer of gold material is associated with an outside surface of the assembly.

The invention also relates to a light emitting device including a shaped member. The shaped member is configured to be juxtaposed one or more body surfaces in a mouth. One or more light emitting members are disposed in the shaped member and are configured to emit energy to the one or more body surfaces for photodynamic therapy. The light emitting device may further include a remote light source operatively coupled to the shaped member via at least one of a fiber optic element, a plurality of internally embedded filaments, or one or more VCSELs, or combinations thereof. The shaped member may include a gel having light diffusing characteristics. The shaped member may be configured to treat a gum line for a disease. The one or more light emitting members may be embedded in the shaped member. The shaped member may be made of a polymer. The shaped member may further include a control operatively coupled to the shaped member to provide a range of intensity or selective operation of the emitted energy. The control may be used to operate the emitted energy in selected pattern or for selective periods of time.

A method of forming a light emitting treatment device is disclosed, including: forming a shell; forming a liner; disposing one or more light emitting members in the liner material; and disposing the liner at least partially in the shell. The shell, liner material and the light emitting members form an assembly. The light emitting members are configured to emit energy at a treatment site.

A product made from the method of forming a light emitting treatment patch is disclosed, including: forming a member; and disposing one or more light emitting members in the member. The member and the one or more light emitting members form an assembly and the light emitting members are configured to emit energy at a treatment site for photodynamic therapy.

A method of using a light emitting treatment device is disclosed, including: identifying an area of treatment on a body surface; applying a surface of a light emitting treatment device to a body surface or tissue. The light emitting treatment device having a liner and one or more light emitting members disposed in the liner. The liner and the light emitting members form an assembly. The light emitting members are configured to emit energy at a treatment site; activating a light transmitting source for a period of time such that the one or more light emitting members emit energy at the area of treatment for photodynamic therapy. The method may further include injecting a drug through the treatment device into the body.

A product made from the process of forming a light emitting treatment device is disclosed, the process including; forming a mold; pouring a material into a recess defined by the mold, the material being configured in a substantially viscous liquid state; disposing one or more filaments in the mold prior to adding the material or embedding the one or more filaments within the material after it has been poured into the recess defined by the mold and curing or drying the material such that the material and the one or more filaments form a treatment device. The process may further include curing the material at an ambient temperature or at an elevated temperature.

The invention also relates to a light emitting treatment device including a member having a thickness. The member is configured to conform to one or more body surfaces. One or more light emitting members are disposed in the member. The member and the one or more light emitting members form an assembly. The one or more light emitting members are configured to emit energy from the assembly for photodynamic therapy of the one or more body surfaces. The member may include one or more light detectors configured to provide surface monitoring. The light emitting treatment device may provide light wavelengths ranging from about 450nm to about 850nm; a light dosage rate ranging from about 0 mW/cm² to about 150 mW/cm²; and a light dose ranging from 0 J/cm² to about 300 J/cm².

The present invention may be used in conjunction with or in relation to inventions disclosed in the following applications, filed on the same date concurrently herewith.

US Patent Applications: M. Biel, Inventor:
Method of Enhancing Photodynamic Therapy by Administering an Immunologic Adjuvant, Serial No. 09/139,861
Dye Treatment Solution and Photodynamic Therapy and Method of Using Same, Serial No. 09/139,866, US Patent No. 6,251,127
Spatial Orientation Grid and Light Sources and Method of Using Same for Medical Diagnosis and Photodynamic Therapy, Serial No. 09/139,862, US Patent No. 6,048,359
Rectangular Laser Irradiation Field Producing Apparatus for Medical Treatment, Serial No. 09/139,480
Methylene Blue and Toluidene Blue Mediated Fluorescence Diagnosis of Cancer, Serial No. 09/139,481, US Patent No. 6,083,487

Still other objects and advantages of the present invention and methods of construction of the same will become readily apparent to those skilled in the art from the following detailed description, wherein only the preferred embodiments are shown and described, simply by way of illustration of the best mode contemplated of carrying out the invention. As will be realised, the invention is capable of other and different embodiments and methods of construction, and its several details are capable of modification in various obvious respects, all without departing from the invention. Accordingly, the drawing and description are to be regarded as illustrative in nature, and not as restrictive.

### Brief Descriptions of the Drawings

Fig. 1 is a plan view of a light-emitting and light diffusing treatment device showing a plurality of radially diverging optical filaments;
Fig. 2 is a plan view of a light-emitting and light diffusing treatment device showing a serpentine optical filament;
Fig. 3 is a cross-section of a light-emitting and light diffusing treatment device constructed of a shell, liner, and embedded optical filament;
Fig. 4 is a plan view of a plurality of VCSELs arrayed in a treatment device coupled to an independent power supply; and
Fig. 5 is a side elevation view of the layers of the treatment device of Figure 4 with an intermediate layer of VCSELs disposed between a heat dissipating layer and a light-diffusing layer;
Fig. 6 is a plan view of a treatment device having VCSELs;
Fig. 7 is a cross-sectional view of an embodiment of the light emitting treatment device;
Fig. 8 is a cross-sectional view of another embodiment of the light emitting treatment device of Figure 6;
Fig. 9 is a cross-sectional view of another embodiment of the light emitting treatment device of Figure 6;
Fig. 10 is a view of the light emitting treatment device disposed on a body surface along with an associated energy source;
Fig. 11 is a shaped device shaped in the form of a mouth piece having energy emitting members;
Fig. 12 is a side view of the shaped device of Figure 11 disposed in a mouth of a patient for PDT;
Fig. 13 is another embodiment of the shaped device;
Fig. 14 is side view of the shaped device of Figure 13 taken along the line of 14-14;
Fig. 15 is a front elevation view of an array of laser diode elements;
Fig. 16 is a diagrammatic side elevation of the laser diode elements of the type disposed in the array of Figure 15; and
Fig. 17 is a diagrammatic side elevation of the array of laser diode elements, lens system, and primary optical fiber utilized for PDT.

### Detailed Description of the Invention

In accordance with this invention, a treatment device 10, 20, 30 such as a patch, pad or shaped article is used to conform to one or more body surface characteristics and to treat the surface or tissue with PDT.

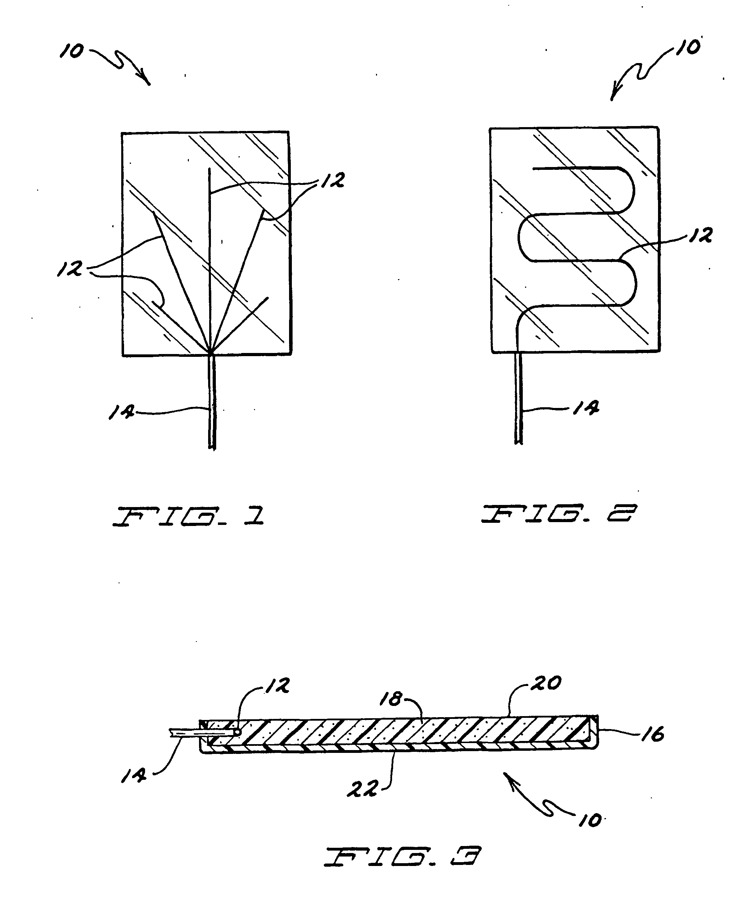

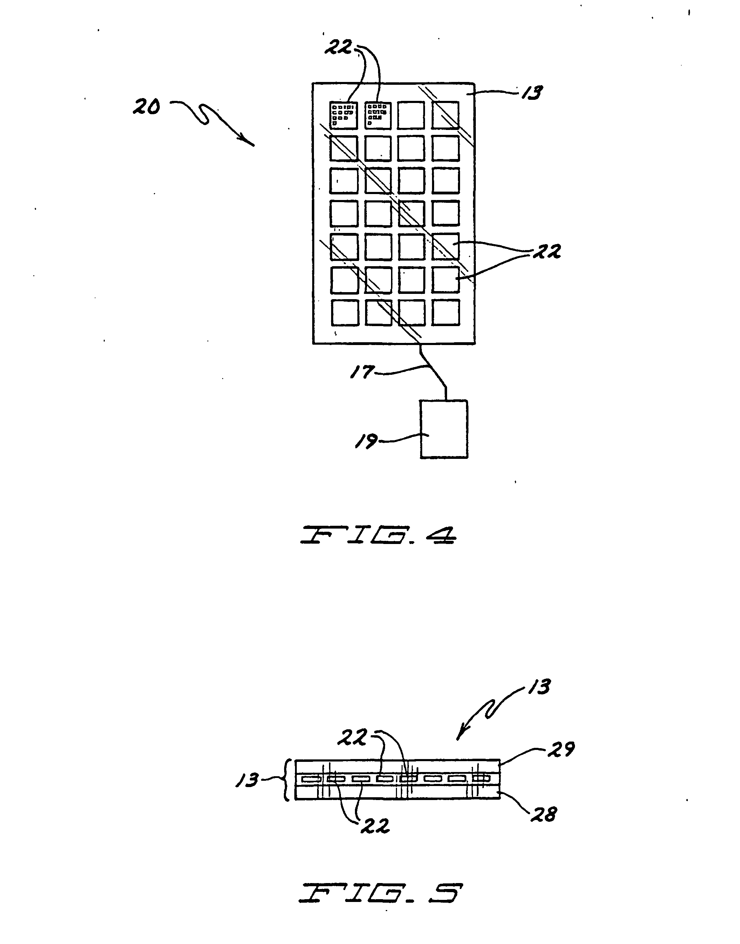

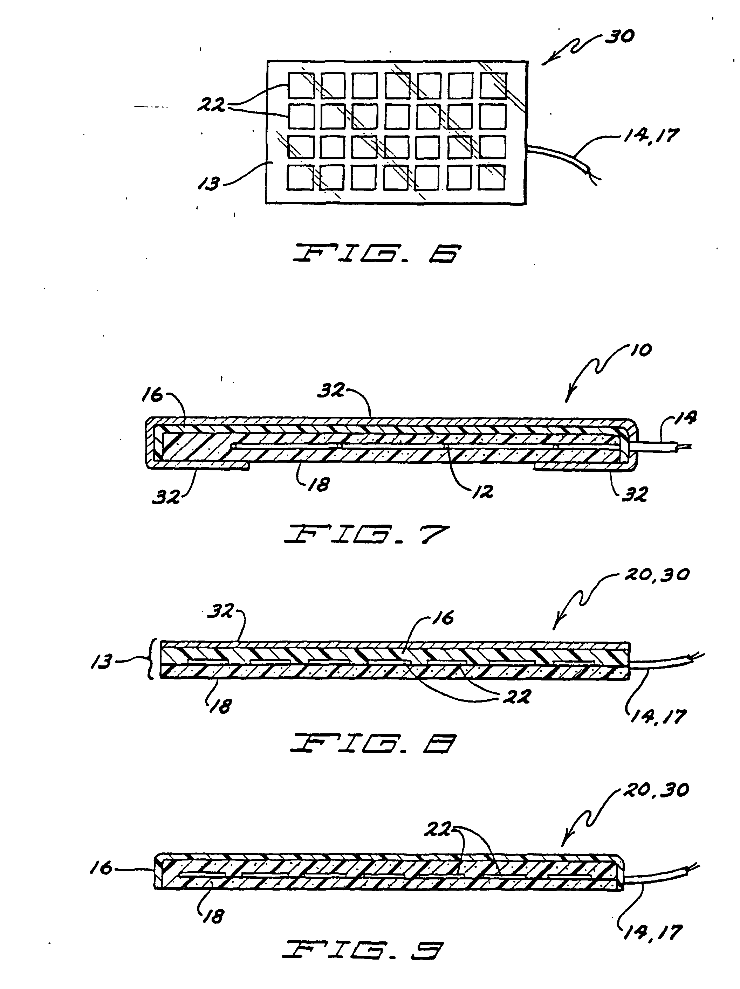

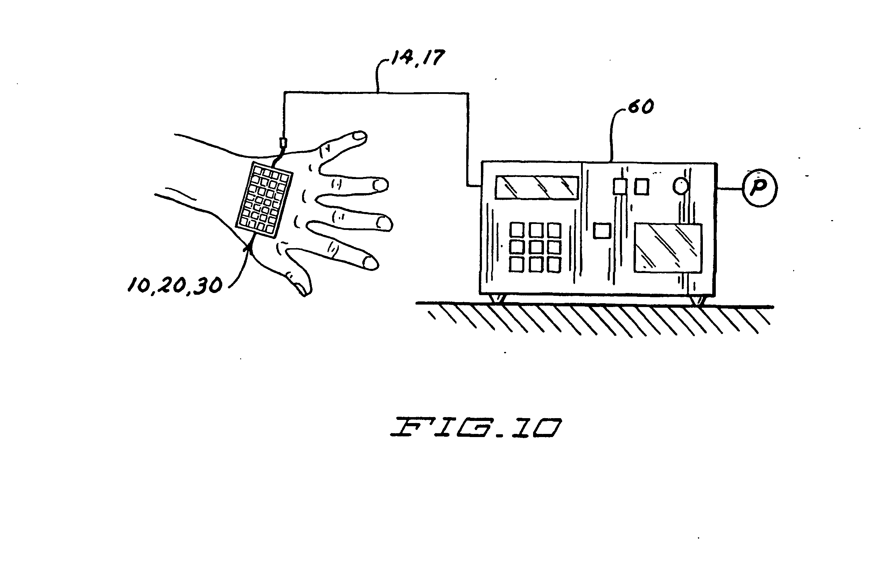

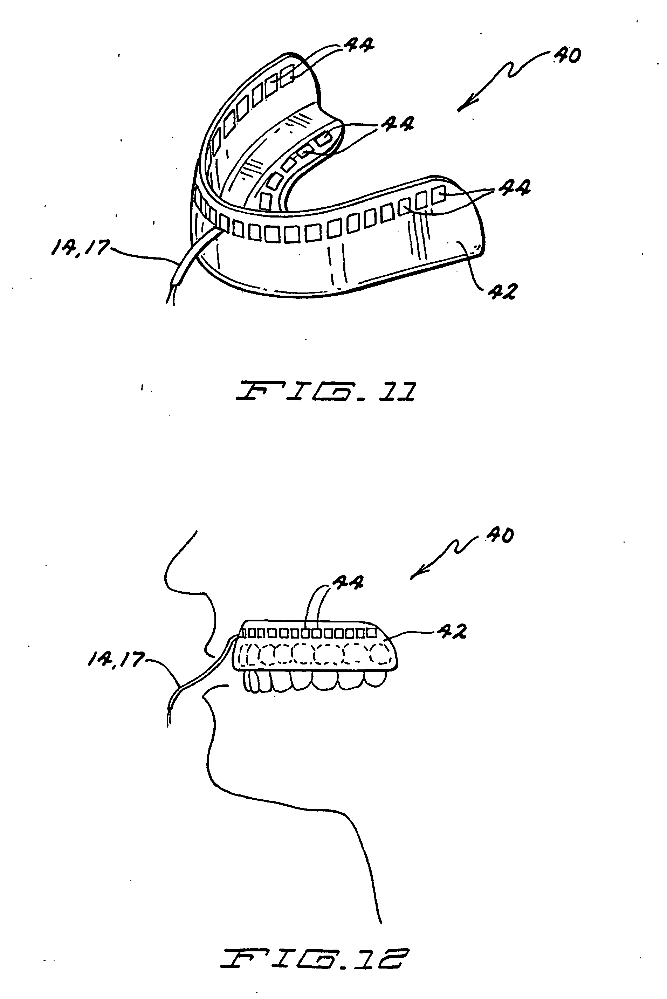

Reference is made to Fig. 1 which illustrates a treatment device 10 which has a plurality of radially diverging optical filaments and provides light-emitting and light diffusing characteristics. The treatment device 10 is used to treat an area of skin or exposed (or exposable) tissue with topical or surface exposure of PDT including generally uniform intensity light energy for long periods. Fig. 2 illustrates another embodiment of a treatment device 10 having a serpentine optical filament 12.

Fig. 3 illustrates a cross-section of a treatment device 10 constructed of a shell 16 having a recessed area, a liner 18 formed in the recessed area, and an optical filament 12 embedded therein. The liner 18 has an exposed surface 20 on the bottom or skin-facing side. Together, the shell 16 and liner 18 define the shape and consistency of the treatment device 10 and form an integral or unitary structure.

A suitable material for the shell 16 and liner 18 is silicone into which one or more optical or light-transmitting filaments 12 may be imbedded. The silicone material generally diffuses the light transmitted from the filaments 12 uniformly over an area of the body being treated.

The shell 16 is preferably made of a cured liquid silicone rubber material such as dimethylsilicone which is sufficiently rigid to support and retain the liner material 18. The shell 16 is preferably made of a highly flexible, compressible, and deformable material having a durometer rating of approximately 20A (Shore units) or less; a tensile strength of about 450 psi minimum; elongation of about 650% minimum; and a tear strength of about 70 ppi minimum. One suitable material is LSR-10 which is liquid silicone rubber available under Product Identification No. 40023 from Applied Silicone Corporation of Ventura, California.

The liner 18 is preferably made of a generally high strength and firm silicone gel composition which is inherently tacky to silicone rubber or skin. The liner 18 has a peel strength of approximately 0.5 ppi (lb./in.) or less from a stainless steel surface; and is hydrophobic and may be cleaned in water and reused without a substantial reduction in its intrinsic tackiness. One suitable material is a silicone gel material that is available under Product Identification No. 40022 from Applied Silicone Corporation of Ventura, California. Both the shell 16 and liner 18 generally appear substantially indistinguishable to the ordinary view of the patient. The shell 16 and liner 18 are generally clear and provide a sufficient texture or internal turbidity for suitable light-diffusing characteristics.

One or more optical filaments 12 may be incorporated in the treatment device 10. The filaments 12 may be formed integral with or operatively connected to an optical fiber 14 and coupled with a remote light source (not shown). The filaments 12 are disposed in a suitable array which uniformly and efficiently disperses the light energy throughout the operative area of the treatment device 10. The array pattern may include radiating spokes or a serpentine configurations as shown in Figures 1-2 or other variations and combinations thereof.

In fabricating one embodiment of the treatment device 10, the shell 16 is first molded and the liner 18 is then poured, while in a highly viscous liquid or syrup-like state, into the recess defined by the shell 16. The filaments 12 may be disposed in the shell 16 prior to adding the liner material 18, or the filaments 12 may be embedded in the liner material 18 after the liquid material has been poured into the recess or during the initial stages of the curing process. The liner material 18 is then allowed to cure or dry. The drying or curing process can occur at ambient temperatures or may be assisted by the application of heat. In general, relatively little surface boundary distinction occurs between the filaments 12 and liner 18. Once cured or dried, the liner 18 bonds securely with the shell 16 and the filaments 12 to form a unitary or integral treatment device 10.

Exposed surface 20 is generally highly tacky to skin and to the opposing surface 22 of the shell 16. If the exposed surface 20 remains in contact with the opposing surface of the shell 22 for an extended period of time, for example, if the treatment device 10 is wrapped circumferentially around an area being treated such as a finger or arm of the patient and adhered to itself, the liner 18 will not permanently bond with the shell 16. Thus, the liner 18 will releasably adhere to the overlapped regions of the shell 16 and the exposed surface 20 is not left behind on the shell 16 when the two surfaces 20, 22 are peeled apart. The shell 16 advantageously acts as a cohesive carrier of the liner material and as an attachable but releasable anchoring substrate for the liner 18. Due to the generally low shear or peel force properties of the liner 18 when in contact with the "reverse" side of the shell 16, the treatment device 10 advantageously exhibits a self-limidng wrapping characteristic. The treatment device 10 cannot be wrapped too tightly or it will naturally unpeel or unwrap itself, thereby preventing inadvertent constriction of blood circulation. The treatment device 10 advantageously remains in place when properly sized for an area of treatment and correct tension is used on a patient. The treatment device 10 advantageously conforms and, if necessary, stretches slightly to contact and cover a desired area for PDT. The treatment device 10 self-adheres to the area contacted by liner 18, and uniformly distributes light energy within the area being treated. The treatment device 10 is generally easily removed from the skin or tissue of the patient without discomfort or adverse affects on the tissue. A hypodermic syringe may be used to inject a therapeutic agent or marker through the treatment device 10 without removal or lifting during treatment. Alternatively, a portion of the treatment device 10 may be peeled away to expose the skin or tissue; to apply other topical medications; or to clean the tissue without removal of the remainder of the treatment device 10.

Fig. 4 illustrates a plurality of array tiles 22 made of VCSELs arrayed in a treatment device 20 which is coupled to an independent power supply 19. A plurality of array tiles 22 made of VCSELs or light emitting diodes (LEDs) are disposed in an array or pattern and adhered to or mounted within a substrate 13 to form a treatment device 20. The treatment device 20 is operatively coupled to an adjustable, self-contained power source 19 such as battery using a conventional wire 17 or cable. The array tiles 22 and VCSELs or LEDs may be wired in series, parallel, or combinations of series and parallel using suitable patterns among adjacent rows or via a peripheral scheme.

The treatment devices 10, 20, 30, 40 may have one or more surface monitor members 21 such as isotropic or anisotropic light detectors or photodetectors. The surface monitor members 21 may be used in combination with a controller to automatically monitor and control characteristics such as light wavelength (nm), light dosage (joules/cm²), and light dosage rate (mW/cm²).

Fig. 5 illustrates a cross-sectional view of an embodiment of a treatment device 20 with an intermediate layer of array tiles 22 made of VCSELs disposed between a heat dissipating layer 29 and a light-diffusing layer 28. The light-diffusing layer 28 is preferably made of a silicone rubber material, and the heat-dissipating layer 29 is preferably made of a material configured to absorb or disperse heat generated by the array tiles 22 made of VSCELs 22. The VCSELs may be disposed in close proximity to one another, or spaced apart to facilitate flexing of the substrate 13. An embodiment of the substrate 13 preferably has dimensions of about 3.0 cm by 1.2 cm and includes 0.3 cm x .3 cm array tiles 22 made of VCSELs.

The array tile 22 made of VCSELs may be any size, shape, or wavelength suitable for a variety of treatment applications. The number of VCSELs per array 22 may be selectively determined and will vary depending upon factors including the required light output (in mW/cm²). The VCSELs are separated or spaced-apart from the tissue surface by a predetermined distance that is dependent upon factors including the incident light energy necessary for treatment, beam divergence, and the thickness or opacity of the light-diffusing layer 28. The separation may be approximately 1-2 mm. The array may be sufficiently flexible or malleable to conform to a variety of body shapes or parts such as the tongue, palate, or cheek, as well as normally exposed skin areas having complex or irregular curvatures or tight curves, such as a patient's arm or leg, finger or toe, heel, wrist, elbow.

Fig. 6 illustrates a treatment device 30 having a plurality of array tiles 22 made of VCSELs.

Fig. 7 illustrates an embodiment of the treatment device 10 with a layer 32 of material such as gold is disposed on one or more surfaces to contain the PDT to a certain area. The layer may be added at the time of treatment depending on the treatment area desired.

Fig. 8 illustrates an embodiment of the treatment device 20, 30 of Figures 4 and 6. An embodiment of a substrate 13 is illustrated that is made of a shell 16 without a recess and a liner 18 formed over the array tiles 22 made of VCSELs and the shell 16. Alternatively, the treatment devices 10, 20, 30 may be made of a substrate 13 formed of one or more materials in a mold. A layer 32 of material such as gold is shown disposed on one surface to contain the PDT to a certain area.

Fig. 9 illustrates another embodiment of the light emitting treatment device 20, 30.

Fig. 10 illustrates a treatment device 10, 20, 30 disposed on a body surface along with an associated energy source and controller 60.

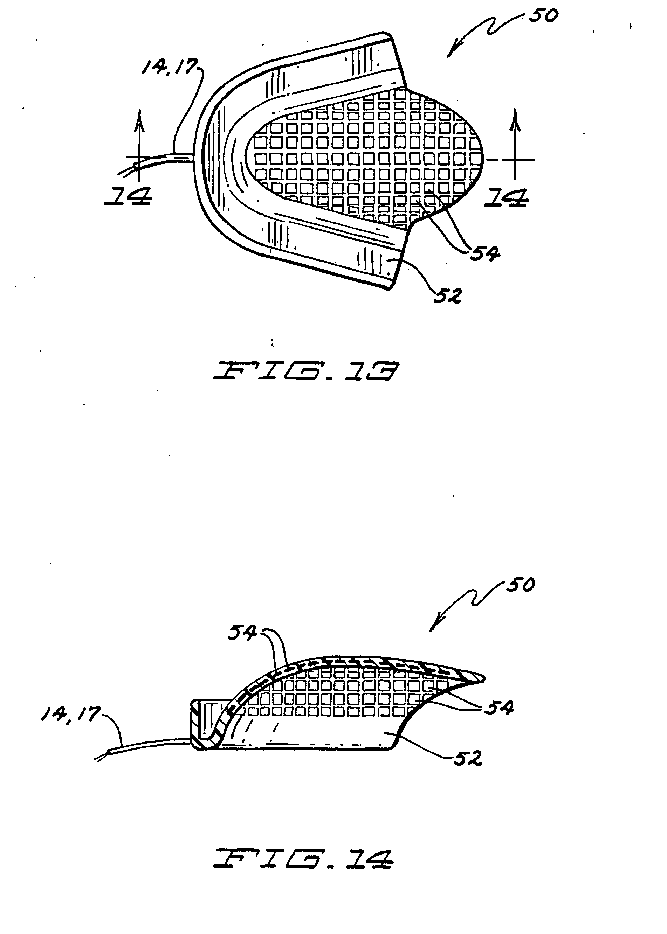

Fig. 11 is a shaped device 40 that is shaped in the form of a mouthpiece 42 and includes energy emitting members 44. One embodiment of the shaped device 40 is in the form a mouthpiece which may be applied over the teeth and gums of a patient for the treatment of cancer. The shaped device 40 may be formed of silicone rubber or a similar light-diffusing material that will conform to a surface area to be treated using PDT. The shaped device 40 is operatively coupled to a remote light source using a fiber optic element or a plurality of internally embedded filaments. Alternatively, the shaped device may itself contain one or more VCSELs disposed in a array tile or pattern and is operatively coupled to a remote power supply. Fig. 12 illustrates the shaped device 40 disposed in a mouth of a patient.

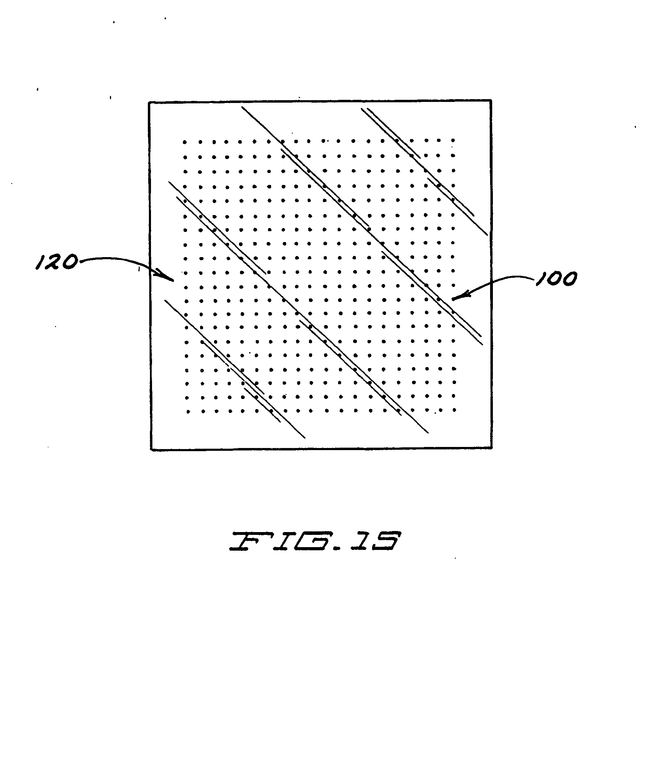

Fig. 13 illustrates another embodiment of the shaped device 50 that is in the form a mouthpiece 52. The mouthpiece 52 includes energy emitting members 54 that are used for PDT of the roof of the mouth. Fig. 14 illustrates the mouthpiece 50 of Figure 13 taken along the line of 14-14.

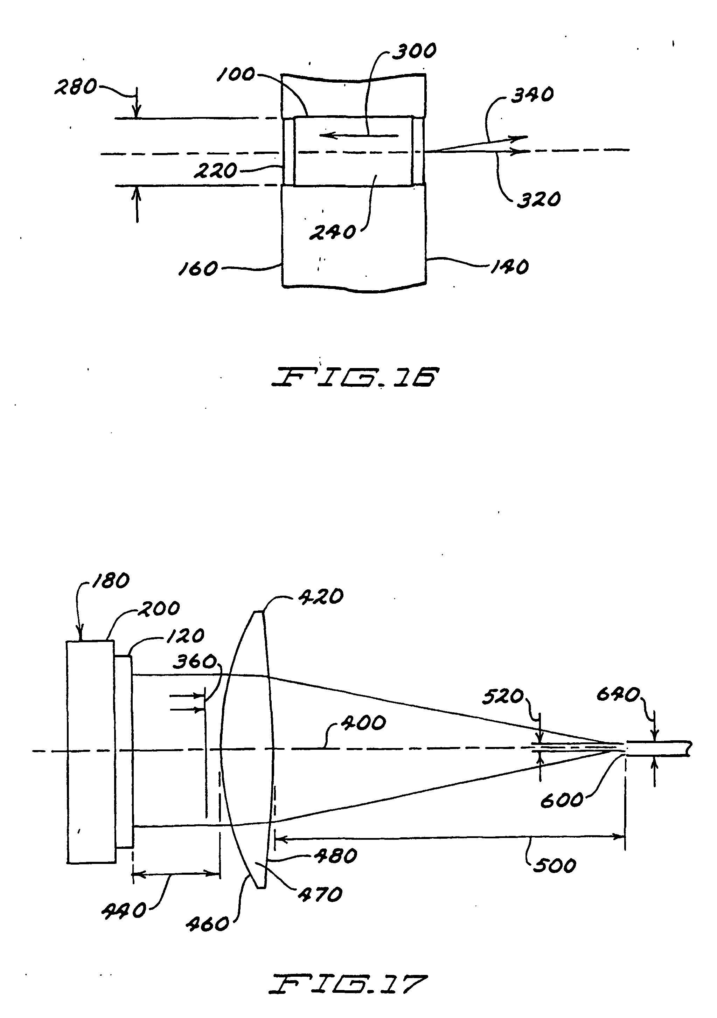

Fig. 15 illustrates an array of laser diode elements that are used for a light emission and transmission system for PDT as an example of a remote light source. A monolithic 20x20 array 120 made of a plurality of laser diode elements 100 has front and rear surfaces 140, 160 that are operatively coupled to a thermal sink 180 for carrying excess heat 200 from the rear surface 160 of the array 120.

Fig. 16 illustrates a laser diode element of the type disposed in the array 120 of Figure 15. Each laser diode element 100 preferably includes a mirror surface 220, a gain medium 240, and an output coupler 260. Energy in the form of a suitable electrically current 300 is input to the gain medium 240 and converted to laser light 320 and emitted from the output coupler 260 substantially normal to the front surface 140. Each laser diode element 100 is nominally cylindrical in shape; approximately 10 microns in diameter 280; and spaced a distance of approximately 100 microns in uniform relationship to adjacent laser diode elements 10. The laser light from each laser diode element 100 has a beam divergence half angle 340 of approximately 3°, and an output power in the range of approximately 0.1 to 5 milliwatts. The aggregate wavefront 360 of the laser diode elements 100 is incoherent with respect to phase, but is substantially plane parallel in the direction of propagation of the optical axis 400, thus impinging upon the lens system 420 as a conjugate in a manner substantially consistent with a beam emanating from a source at an infinite distance.

Lens system 420 shares the same optical axis 400 as the array 120 and is separated from the array 12 by a distance 440 and separated from the focal point 520 by a distance 500. The lens system 420 has front and rear refractive surfaces 460, 480, respectively, defined by the refractive medium 470. The curvatures of the refractive surfaces 460, 480 and the focal distance 500 are optimally selected to condense or converge the aggregate wavefront 360 to a minimal focal area 520 at the "launch face" of the primary optical fiber 600, and optimally selected to meet the numerical aperture requirements of that primary optical fiber 600. For the embodiment described, the focused beam area will underfill an optical fiber of approximately 400 micron core size 640.

The primary optical fiber 600 transmits the focused light energy to a light delivery mechanism (not shown), which consists at least of a disposable optical fiber operatively connected to a beam-directing instrument suitable for the specific treatment modality of application. Fig. 17 illustrates an array of laser diode elements, lens system, and primary optical fiber utilised to emit and transmit laser light energy for PDT as an example of a remote light source.

The light delivery mechanism is provided to the medical professional in a sterile form, and is removed from its packaging in preparation of a PDT session, and coupled prior to treatment to the primary optical fiber 600 proximate to its enclosure or housing (not shown) using a suitable focusing or butt coupling.

The above described embodiments of the invention are merely descriptive of its principles and are not to be considered limiting. Further modifications of the invention herein disclosed will occur to those skilled in the respective arts and all such modifications are deemed to be within the scope of the invention as defined by the following claims.

## Claims

1. A light emitting treatment device (10) comprising:
a flexible substrate element comprising a gel material and adapted to be used in association with a treatment field;
one or more light emitting members (12) disposed within the substrate element, said one or more light emitting members (12) providing a light dosage rate to the treatment field at a value between about 0 mW/cm² to about 150 mW/cm²;
a heat dissipating layer (29) for transferring heat generated by the one or more light emitting members (12) away from the treatment field **characterised by**
an outer layer (32) disposed upon the substrate element for restricting the area of the treatment field exposed to light from the treatment device.

2. The light emitting treatment device (10) of claim 1 wherein the substrate element is suitable for being placed in touching contact with a patient during a photodynamic therapy.

3. The light emitting treatment device (10) of claim 2 wherein the substrate element is shaped to be received into a mouth during the photodynamic therapy.

4. The light emitting treatment device (10) of claim 1 wherein the one or more light emitting members (12) are disposed in a substantially uniform array.

5. The light emitting treatment device (10) of claim 1 wherein the one or more light emitting members (12) include one or more light emitting members (12) within the group including VCSELs and LEDs.

6. The light emitting treatment device (10) of claim 1 wherein the one or more light emitting members (12) are fiber optic elements fed from a light source remote from the treatment field.

7. The light emitting treatment device (10) of claim 1 wherein a portion of the outer layer (32) is disposed between the light emitting treatment device (10) and the treatment field, said outer layer (32) being selectively configured in relation to an area within the treatment field.

8. The light emitting treatment device (10) of claim 1 wherein the outer layer (32) is formed from gold.

9. The light emitting treatment device (10) of claim 1 wherein the outer layer (32) is capable of being added to the device at the time of treatment.

10. A kit comprising the light emitting treatment device (10) of claim 1 and an outer layer (32) capable of being attached to the treatment device to restrict the area of the treatment field exposed to light from the treatment device.

11. The light emitting treatment device (10) of claim 1 further comprising:
an adhesive element for adhesively securing the treatment device at the treatment field during a photodynamic therapy.

12. The light emitting treatment device (10) of claim 1 wherein the substrate is optically diffusive.

13. The light emitting treatment device (10) of claim 1 further comprising:
a controller device operatively coupled to the one or more light emitting members (12), said controller device selectively controlling the light intensity in response to the feedback signal from one or more light detector elements.

## Patentansprüche

1. Licht emittierende Einrichtung (10), umfassend:
ein flexibles Substratelement, das ein Gelmaterial umfasst und ausgeführt ist, in Verbindung mit einem Behandlungsfeld verwendet zu werden;
ein oder mehrere Licht emittierende Elemente (12), die innerhalb des Substratelements angeordnet sind, wobei das eine oder die mehreren Licht emittierenden Elemente (12) dem Behandlungsfeld eine Lichtdosisleistung mit einem Wert zwischen etwa 0 mW/cm² bis etwa 150 mW/cm² bereitstellen;
eine Wärme ableitende Schicht (29) zum Transportieren von Wärme, die von dem einen oder mehreren Licht emittierenden Elementen (12) erzeugt wird, vom Behandlungsfeld fort, **gekennzeichnet durch**
eine äußere Schicht (32), die auf dem Substratelement angeordnet ist, zum Beschränken der Fläche des Behandlungsfeldes, die Licht von der Einrichtung ausgesetzt ist.

2. Licht emittierende Einrichtung (10) nach Anspruch 1, wobei das Substratelement geeignet ist, während einer photodynamische Therapie in Berührungskontakt mit einem Patienten platziert zu werden.

3. Licht emittierende Einrichtung (10) nach Anspruch 2, wobei das Substratelement geformt ist, um während der photodynamischen Therapie in einem Mund aufgenommen zu sein.

4. Licht emittierende Einrichtung (10) nach Anspruch 1, wobei das eine oder die mehreren Licht emittierenden Elemente (12) in einer im Wesentlichen gleichförmigen Anordnung angeordnet sind.

5. Licht emittierende Einrichtung (10) nach Anspruch 1, wobei das eine oder die mehreren Licht emittierenden Elemente (12) ein oder mehrere Licht emittierende Elemente (12) innerhalb der Gruppe beinhalten, die VCSELs und LEDs beinhaltet.

6. Licht emittierende Einrichtung (10) nach Anspruch 1, wobei das eine oder die mehreren Licht emittierenden Elemente (12) faseroptische Elemente sind, die von einer Lichtquelle versorgt werden, die vom Behandlungsfeld entfernt ist.

7. Licht emittierende Einrichtung (10) nach Anspruch 1, wobei ein Abschnitt der äußeren Schicht (32) zwischen der Licht emittierenden Einrichtung (10) und dem Behandlungsfeld angeordnet ist, wobei die äußere Schicht (32) selektiv in Relation auf eine Fläche innerhalb des Behandlungsfeldes konfiguriert ist.

8. Licht emittierende Einrichtung (10) nach Anspruch 1, wobei die äußere Schicht (32) aus Gold gebildet ist.

9. Licht emittierende Einrichtung (10) nach Anspruch 1, wobei die äußere Schicht (32) in der Lage ist, der Einrichtung zur Zeit der Behandlung hinzugefügt zu werden.

10. Satz, umfassend die Licht emittierende Einrichtung (10) nach Anspruch 1 und eine äußere Schicht (32), die in der Lage ist, an der Einrichtung angebracht zu werden, um die Fläche des Behandlungsfeldes zu beschränken, die Licht von der Einrichtung ausgesetzt ist.

11. Licht emittierende Einrichtung (10) nach Anspruch 1, ferner umfassend:
ein adhäsives Element zum adhäsiven Befestigen der Einrichtung am Behandlungsfeld während einer photodynamischen Therapie.

12. Licht emittierende Einrichtung (10) nach Anspruch 1, wobei das Substrat optisch diffusiv ist.

13. Licht emittierende Einrichtung (10) nach Anspruch 1, ferner umfassend:
ein Steuergerät, das operativ mit dem einen oder den mehreren Licht emittierenden Elementen (12) gekoppelt ist, wobei das Steuergerät die Lichtintensität in Reaktion auf das Rückkopplungssignal von einem oder mehreren Lichtdetektorelementen selektiv steuert.

## Revendications

1. Dispositif de traitement émettant de la lumière (10) comprenant :
un élément de substrat flexible comprenant un matériau de gel et adapté pour une utilisation en association avec un champ de traitement ;
un ou plusieurs éléments émettant de la lumière (12) disposés au sein de l'élément de substrat, lesdits un ou plusieurs éléments émettant de la lumière (12) fournissant un débit de dose de lumière au champ de traitement d'une valeur comprise entre environ 0 mW/cm2 et 150 mW/cm2 ;
une couche de dissipation de la chaleur (29) pour transférer la chaleur générée par l'un ou plusieurs éléments émettant de la lumière (12) à l'opposé du champ de traitement, **caractérisé par**
une couche externe (32) disposée sur l'élément de substrat pour restreindre la surface du champ de traitement exposée à la lumière du dispositif de traitement.

2. Dispositif de traitement émettant de la lumière (10) selon la revendication 1, dans lequel l'élément de substrat est approprié pour être placé en contact direct avec in patient au cours d'une thérapie photodynamique.

3. Dispositif de traitement émettant de la lumière (10) selon la revendication 2, dans lequel l'élément de substrat est formé pour être reçu dans une embouchure au cours de la thérapie photodynamique.

4. Dispositif de traitement émettant de la lumière (10) selon la revendication 1, dans lequel l'un ou plusieurs des éléments émettant de la lumière (12) sont disposes dans un groupement essentiellement uniforme.

5. Dispositif de traitement émettant de la lumière (10) selon la revendication 1, dans lequel l'un ou plusieurs des éléments émettant de la lumière (12) comprennent un ou plusieurs éléments émettant de la lumière (12) au sein du groupe comprenant des VCSEL et des DEL.

6. Dispositif de traitement émettant de la lumière (10) selon la revendication 1, dans lequel l'un ou plusieurs des éléments émettant de la lumière (12) sont des éléments de fibre optique alimentés d'une source de lumière distante du champ de traitement.

7. Dispositif de traitement émettant de la lumière (10) selon la revendication 1, dans lequel une partie de la couche externe (32) est disposée entre le dispositif de traitement émettant de la lumière (10) et le champ de traitement, ladite couche externe (32) étant configurée de manière sélective par rapport à une surface au sein du champ de traitement.

8. Dispositif de traitement émettant de la lumière (10) selon la revendication 1, dans lequel la couche externe (32) est formée à partir d'or.

9. Dispositif de traitement émettant de la lumière (10) selon la revendication 1, dans lequel la couche externe (32) est capable d'être ajoutée au dispositif au moment du traitement.

10. Kit comprenant le dispositif de traitement émettant de la lumière (10) selon la revendication 1 et une couche externe (32) capable d'être fixée au dispositif de traitement pour restreindre la surface du champ de traitement exposée à la lumière du champ de traitement.

11. Dispositif de traitement émettant de la lumière (10) selon la revendication 1 comprenant en outre :
un élément adhésif pour sécuriser de manière adhésive le dispositif de traitement au champ de traitement au cours de la thérapie photodynamique.

12. Dispositif de traitement émettant de la lumière (10) selon la revendication 1, dans lequel le substrat présente une diffusion optique.

13. Dispositif de traitement émettant de la lumière (10) selon la revendication 1, comprenant en outre :
un dispositif de commande couplé de manière fonctionnelle à l'un ou plusieurs des éléments émettant de la lumière (12), ledit dispositif de commande commandant de manière sélective l'intensité lumineuse en réponse au signal en retour d'un ou de plusieurs éléments de détection de lumière.
